# EUROPEAN PATENT APPLICATION

(11) **EP 0 930 069 A2**
(43) Date of publication of application: **21.07.1999**
(21) Application number: 99300201.3
(22) Date of filing: 12.01.1999
(51) Int. Cl.: A61K 31/52

(54) **Compositions for the reduction of scarring**

(30) Priority: 13.01.1998 GB 9800640
(71) Applicant: Johnson & Johnson Medical Ltd., Edinburgh EH2 4NH (GB)
(72) Inventor: Grady, Michael William, Burley-in-Wharfedale, W.Yorks. LS29 7LP (GB); Bloor, Stephen, Chorley, Lancashire PR6 7QL (GB)
(74) Representative: James, Anthony Christopher W.P.

(57) **Abstract**

The invention provides the use of one or more substances selected from the group consisting of (a) an adenylyl cyclase agonist, (b) cyclic 3', 5'-adenosine monophosphate (cAMP) and salts and derivatives thereof, and (c) phosphodiesterase inhibitors for the preparation of a composition for the reduction of wound scarring. The substances act by increasing cAMP levels in the wound, thereby reducing cell proliferation resulting in reduced scarring. Preferred substances include forskolin, dybutyryl cAMP, lisofylline, and isobutyl methyl xanthine (IBMX).

## Description

The present invention relates to the use of certain substances for the preparation of compositions for use in the reduction of scar formation during the healing of wounds in the human or animal body.

Cyclic 3', 5'-adenosine monophosphate (cyclic AMP or cAMP) has been the subject of a great number of studies. Cyclic AMP appears to be involved in the regulation of functional metabolism of a large variety of tissues, and also has a role in hormone action. It is also known that body enzymes such as phosphodiesterases inactivate cAMP by converting it to the 5'-adenosine monophosphate.

US-A-3849553 describes a method for treating psoriasis in humans comprising the administration of a cAMP derivative. There is no suggestion that the cAMP derivative could reduce scarring of acute wounds.

US-A-3978213 describes compositions for dermatological and cosmetic use containing cAMP and salts thereof and optionally also containing a phosphodiesterase inhibitor such as the theophylline. The compositions are alleged to exhibit anti-phlogistic, skin softening and skin elasticity and moisture-increasing activities. There is no suggestion that the compositions could reduce scar formation in acute wound healing.

It has now been found that increasing levels of cAMP can reduce fibroblast proliferation *in vitro*. This is expected to result in reduced scar formation in the healing of acute wounds.

Accordingly, the present invention provides the use of one or more substances selected from the group consisting of (a) adenylyl cyclase agonists, (b) cyclic 3', 5' -adenosine monophosphate (cAMP) and salts and derivatives thereof, and (c) phosphodiesterase inhibitors, for the preparation of a composition for the reduction of wound scarring.

Each of the substances (a), (b), and (c) will have the affect of increasing effective cAMP levels in a wound. For example, the adenylyl cyclase agonists stimulate the production of cAMP in mammalian cells. The cAMP and salts thereof directly increase the concentration of cAMP in the wound bed, and the cAMP derivatives are selected to have similar activity. Finally, the phosphodiesterase inhibitors reduce the rate at which cAMP is broken down *in vivo* by natural phosphodiesterase enzymes. Two or more of the substances (a), (b) and (c) may be used, and the two or more substances may even be packaged separately as a combined preparation for simultaneous, separate or sequential administration.

Preferably, the adenylyl cyclase agonist comprises Forskolin, or another activator derived from *Eoleus Forskolii,*

Preferably, the cAMP derivative has formula 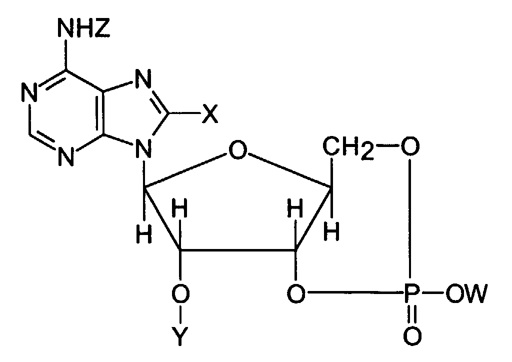 wherein Y and Z are each selected from a group consisting of H or butyryl; X is selected from a group consisting of H, -Br, -SCH₃ -SH, OH, and -NH₂, and W is H or a pharmaceutically acceptable cation such as sodium, potassium, zinc or ammonium.

The term "cAMPderivative" generally signifies a chemical derivative, such as an amide or an ester. The preferred derivatives exhibit similar bio chemical activity to cAMP, but are preferably broken down much more slowly than cAMP by naturally occurring phosphodiesterase enzymes. The particularly preferred cAMP derivative of this type is dybutyryl cAMP.

Preferably, the phosphodiesterase inhibitor is selected from the group consisting of isobutyl methyl xanthine (IBMX), papaverine, xanthine, rolipram, theophylline, caffein, pentoxifylline, lisofylline, type 3 and type 4 phosphodiesterase inhibitors and beta adrenergic agonists such as propranolol or salmeterol. A preferred phosphodiesterase inhibitor is the broad spectrum phosphodiesterase inhibitor IBMX.

A particularly preferred phosphodiesterase inhibitor is lisophylline. Lisofylline has previously been studied as a haematopoiesis modulator and lysophosphatidic acid acyl-CoA:acyltransferase inhibitor by A. Graul *et al.* In Drugs Future Vol. 22(5), pages 492-498 (1997), the entire content of which is incorporated herein by reference. Lisofylline has also been proposed for the prevention of tissue damage by hypoxia in WO95/13075, the entire content of which is also incorporated herein by reference.

The compositions used in the present invention may be formulated as any standard wound treatment known in the art, such as a solid wound dressing or a semi-solid gel or ointment comprising these substances in an aqueous form. Suitable solid wound dressing materials include, for example, woven or non-woven textile webs having the substances coated thereon, or various slow release formulations such as collagen sponges having the substances impregnated therein. The semi-solid gel or ointment preferably comprises in a aqueous vehicle comprising 0.1-2.0% of a gelling agent such as a sodium carboxymethyl cellulose, and optionally containing an antiseptic and standard pharmaceutical excipients.

The concentration of the active substances in the compositions according to the present invention will depend on the intended mode of administration and the duration of release. It appears that the substances are active in a concentration range of from 1 micromolar to 10 millimolar, preferably 10 micromolar to 1 millimolar, and more preferably 20 micromolar to 500 micromolar, and any gel, ointment or solid or slow release formats should preferably be adapted to provide similar concentrations at the surface of the wound in use. For example, medicaments preferably contain the substances at a concentration range of from 0.001 to 5.0% w/v, more preferably 0.01 to 1% wlv.

The present invention also provides a method of treating a wound to reduce scarring during wound healing, the method comprising applying to the wound a composition containing an effective amount of one or more substances selected from the group consisting of (a) an adenylyl cyclase agonist (b) cyclic 3', 5'-adenosine monophosphate (cAMP) and salts and derivatives thereof, and (c) a phosphodiesterase inhibitor. Preferably, the composition is applied to acute wounds, such as surgical incisions. Preferably, the composition is applied within 24 hours of wounding, more preferably within 6 hours of wounding

The present invention will now be described in more detail with reference to the accompanying drawings, in which:-
Figure 1 shows graphically the effect of prior incubation (5hr) with cAMP raising agents on platelet derived growth factor (PDGF) stimulated fibroblast proliferation;
Figure 2 shows the effect of varying concentrations of forskolin on PDGF stimulated fibroblast proliferation at varying concentration in 10% foetal bovine serum (FBS);
Figure 3 shows graphically the effect of varying concentrations of dibutyryl cAMP on fibroblast proliferation at different concentrations of foetal bovine serum (FBS).

### Procedure 1

The effect of substances for use in the present invention on fibroblast cell proliferation *in vitro* was assessed as follows.

Forskolin F6886, dibutyryl cyclic AMP D0627 and IBMX 17018 were all obtained from the Sigma Chemical Company, U.K. Platelet derived growth factor (PDGF) was obtained from R & D Systems, of Abingdon OX14 3YS, U.K.. Methylene blue M/4870/45 was obtained from Fisons Limited, Loughborough LE11 ORG, U.K..

Adult dermal human fibroblasts from foreskin were grown in the presence of Dulbeccos Modified Eagles Medium (DMEM) containing 10% foetal bovine serum (FBS) in cell culture dishes. When the cells were 90% confluent the medium was aspirated and replaced with 10 mls of sterile phosphate buffered saline. This was aspirated off and replaced by 2 mls of sterile trypsin which allows the cells to detach from the culture dish. 8 mls of 10% FBS-DMEM was added to the plate to de-activate the trypsin.

The resulting fluid was spun at 2000 rpm for 8 minutes. The supernatant was aspirated off and replaced with sterile 0% FBS-DMEM. The cell pellet was dislodged and gently mixed using a sterile 5 ml pipette. 100 µl of the cell suspension containing 5000 cells was added to each well of a 96 well culture dish. The cells were allowed to attach overnight in 10% FBS-DMEM. The medium was then replaced with 100 ml of 0% or 2% or 5% FBS-DMEM.

The test compounds (IBMX, Forskolin and dibutyryl cyclic AMP) were prepared as stock concentration in ethanol, FBS and DMSO (dimethyl sulfoxide), respectively. Forskolin 10 mg/ml. = 25 mM, IBNX 5 mg/ml = 23 mM, and dibutyryl cyclic AMP 10 mg/ml = 20 mM.

The final concentrations of each compound range from 100-0.1 µM in the appropriate serum or serum containing DMEM. In some experiments the cells were incubated with 100 ml of PDGF at 50 ng/ml before the immediate addition of each cAMP raising agent to achieve the desired concentration. In other experiments the cAMP raising agents were allowed to incubate for a period of five hours with the fibroblasts before adding 100 ml of PDGF at 15 ng/ml.

After addition of the test agents, the cells were allowed to proliferate for three days in a 37°C incubator before the amount of cell proliferation was quantified by methylene blue assay as detailed by M. Elliot and N. Auersperg in Biotechnic & Histochemistry, Vols 68(1), 1993 pages 113-117.

Platelet derived growth factor (PDGF) was used as a positive control in these experiments as a concentration 15 ng/ml diluted in 0% FBS-DMEM.

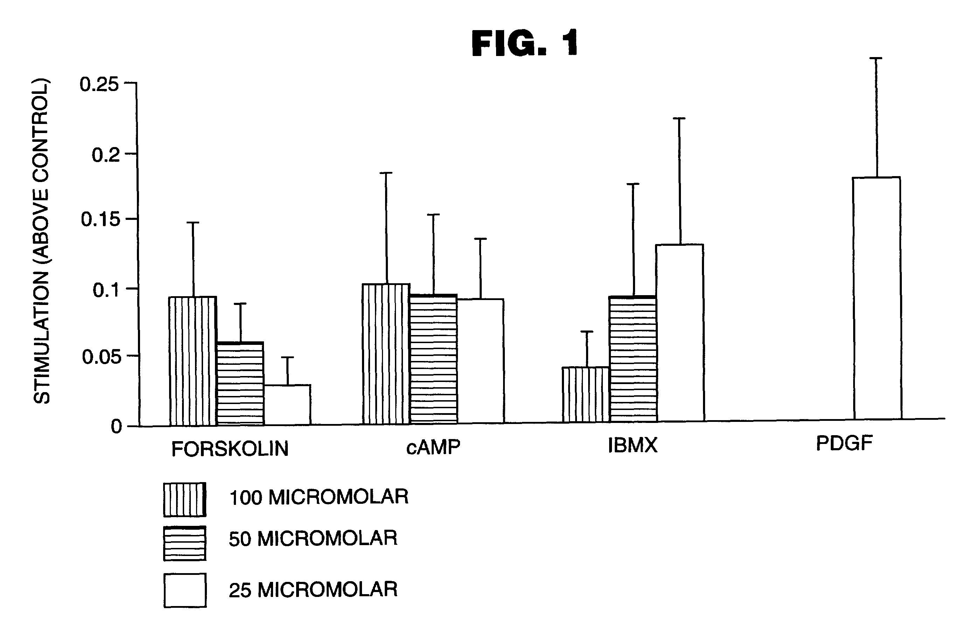

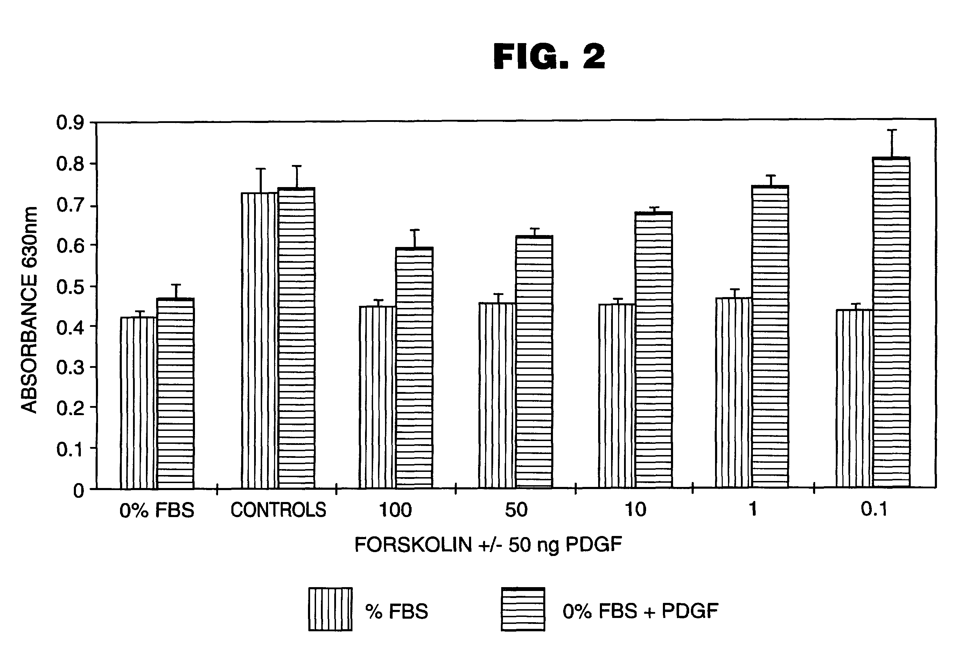

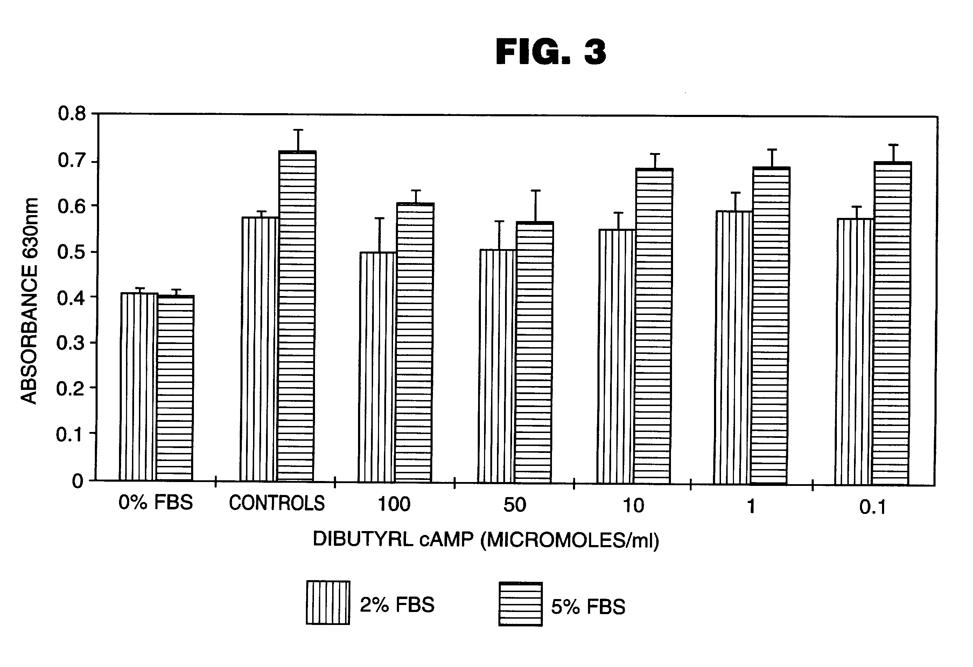

Referring first to the data as shown in Figure 1, it can be seen that PDGF stimulated fibroblast proliferation is reduced at concentrations of 25, 50 and 100 mM of forskolin, dibutyryl cAMP and IBMX. Likewise, it can be seen from Figures 2 and 3 that forskolin and dibutyryl cAMP both exhibit a concentration-dependent effect on PDGF simulated fibroblast proliferation *in vitro.*

Without wishing to bound by any theory, it is thought that cAMP reduces PDGF stimulated cell proliferation by phosphorylating a mitogen activated protein kinase (APK) which is crucial for cell proliferation to occur in response to serum or growth factors. It is further expected that this effect will result in significantly reduced scarring in the event that cyclic AMP levels are elevated in wounds during healing.

### Example 1

A series of topical gel ointment compositions containing varying amounts of IBMX suitable for application to an acute wound to reduce scarring is prepared by mixing the following components at ambient temperature:-
0.05%, 0.25%, 1.0% and 5.0% w/v of IBMX
25% w/v of propylene glycol
3% w/v sodium alginate
1% w/v carboxymethyl cellulose
0.35% w/v hydroxyethyl cellulose
0.3% w/v NaCI
to 100% distilled water.

The resulting clear, gel ointment is intended for application directly to a wound if prepared under sterile conditions.

### Example 2

A series of topical gel ointment compositions were prepared as described in Example 1, but with lisophylline in place of the IBMX. The resulting clear, gel ointment is intended for application directly to a wound if prepared under sterile conditions.

The above procedure and examples have been described for the purpose of illustration only. Many other embodiments falling within the scope of the accompanying claims will be apparent to the skilled reader.

## Claims

1. Use of one or more substances selected from the group consisting of (a) adenylyl cyclase agonists, (b) cyclic 3', 5' - adenosine monophosphate (cAMP) and salts and derivatives thereof, and (c) phosphodiesterase inhibitors, for the preparation of a composition for the reduction of wound scarring.

2. Use according to claim 1, wherein the adenylyl cyclase agonist comprises forskolin.

3. Use according to claim 1 or 2, wherein the cAMP derivative or salt has formula 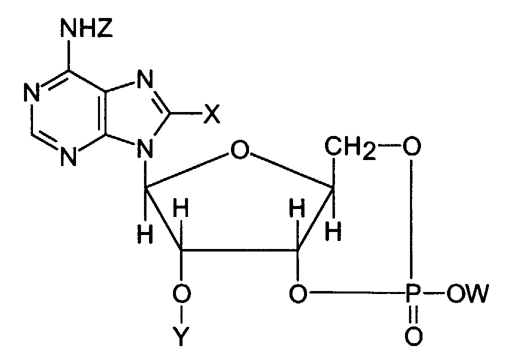 wherein Y and Z are each selected from the group consisting of H or butyryl; X is selected from the group consisting of H, -Br, -SCH₃, -SH, -OH and -NH₂; and W is H or a pharmaceutically acceptable cation.

4. Use according to claim 3, wherein the cAMP derivative comprises dibutyryl cAMP or a salt thereof.

5. Use according to any preceding claim, wherein the phosphodiesteraseinhibitor is selected from isobutyl methyl xanthine (IBMX), papaverene, a xanthine, rolipram, theophylline, caffein, pentoxifylline, lisofylline, a phosphodiesterase 3 inhibitor, a phosphodiesterase 4 inhibitor or a beta-adrenergic agonist.

6. Use according to claim 5, wherein the phosphodiesterase inhibitor comprises isobutyl methyl xanthine (IBMX).

7. Use according to claim 5, wherein the phosphodiesterase inhibitor comprises lisofylline.

8. Use according to any preceding claim, wherein said composition is a solid wound dressing material.

9. Use according to any claims 1 to 6, wherein said composition is a semisolid gel or ointment comprising said substance in an aqueous vehicle.
